# EUROPEAN PATENT APPLICATION

(11) **EP 4 201 923 A1**
(43) Date of publication of application: **28.06.2023**
(21) Application number: 21217331.4
(22) Date of filing: 23.12.2021
(51) Int. Cl.: C07D 211/86, C07D 401/06, A61P 31/04, A61P 35/00, A61P 35/02, A61K 31/4412, A61K 31/4439, A61K 31/4545

(54) **5-SUBSTITUTED 2,3-DIHYDROPYRIDIN-4-ONES AND PROCESS FOR PREPARING THE SAME**

(71) Applicant: Karl-Franzens-Universität Graz, 8010 Graz (AT)
(72) Inventor: Seebacher, Werner, 8044 Graz (AT)
(74) Representative: Schwarz & Partner Patentanwälte GmbH

(57) **Abstract**

The present invention relates to a process for preparing a compound of formula (I) 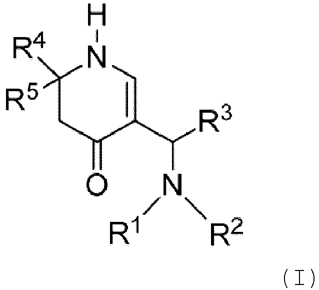 comprising a step of reacting a compound of formula (II) 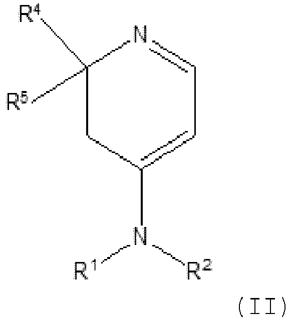 or a salt thereof
with an aldehyde compound of formula (III)

R³-CHO (III)

as well as a compound of formula (I) and its use as medicament, in particular for the treatment of infections or cancer.

## Description

### TECHNICAL FIELD

The present invention relates to a process for preparing a 2,3-dihydropyridin-4-one and novel 2,3-dihydropyridin-4-ones as well as uses thereof.

### BACKGROUND ART

2,3-Dihydropyridin-4-ones are valuable synthons and intermediates for reactions like cycloadditions, electrophilic substitutions, and they can be functionalized on the N and the O atom.

2,3-Dihydropyridin-4-ones (or 2,3-dihydropyridin-4(1H)-ones or alternatively 2,3-dihydro-4-pyridones) are electron-rich compounds and as such valuable starting points (synthons) for multiple reactions (Niphakis MJ et al. J. Org. Chem. 2010, 75 6793-6805). For example, 5,6-dihydro-4-pyridones were shown to react in intramolecular enone [2+2] photocycloaddition reactions (Brimioulle R et al. Science 2013, 342, 840).

There is a need for new 2,3-dihydropyridin-4-ones and processes for preparing said compounds allowing for more structural variability in these valuable synthons for further reactions.

Moreover, the chemically related dihydropyridin-2-ones are described to exhibit pharmaceutical activities. For example, WO 2015134699 A1 discloses tetrazolone-substituted dihydropyridinone compounds, describes their property as monoacylglycerol acyltransferase type 2 (MGAT2) inhibitors and suggested their use in treatment of diabetes, obesity, dyslipidemia and related conditions.

The 2,3-dihydrophydridin-4-one is a partial structure found in a natural product, cenocladamide (Santos CCF et al. Med. Chem. Comm. 2017, 8 755-766), an alkaloid with anticancer potency and deterrent activity against ants.

It is therefore an object of the present invention to provide alternative processes for preparing new 2,3-dihydropyridin-4-ones, which are of high interest both as synthons for further chemical processing as well as due to their potential biological properties.

### SUMMARY OF THE INVENTION

The present invention relates to a process for preparing a compound of formula (I) 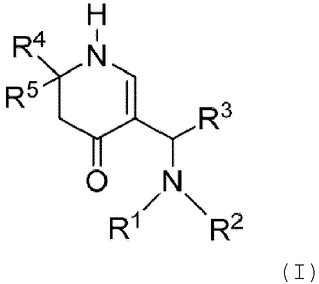 comprising a step of reacting a compound of formula (II) 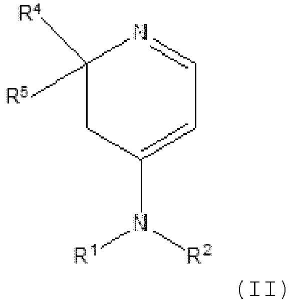 or a salt thereof
with an aldehyde compound of formula (III)

R³-CHO (III)

wherein R¹ and R² form together with the nitrogen to which they are bound a substituted or unsubstituted 5-, 6- or 7-membered heterocyclyl group, or
wherein R¹ and R² are independently from each other selected from the group consisting of hydrogen, unsubstituted or substituted C₁-C₁₀-alkyl, and unsubstituted or substituted C₆-C₁₄-aryl,
wherein R³ is selected from the group of unsubstituted or substituted C₁-C₁₀-alkyl, unsubstituted or substituted C₃-C₁₀-cycloalkyl, unsubstituted or substituted C₆-C₁₄-aryl, and unsubstituted or substituted 5-, 6- or 7-membered heteroaryl, and
wherein R⁴ and R⁵ are independently from each other a substituted or unsubstituted C₁-C₁₀-alkyl group,
wherein R⁴ is selected from the group consisting of substituted or unsubstituted C₁-C₁₀-alkyl, preferably a branched C₃-C₁₀-alkyl group, and unsubstituted or substituted C₃-C₁₀-cycloalkyl and R⁵ is hydrogen,
or wherein R⁴ and R⁵ form together with the carbon to which they are bound a substituted or unsubstituted C₃-C₁₀-cycloalkyl.

It was surprisingly found that a 4-amino substituted 2,3-dihydropyridine of the formula (II) (or the corresponding tetrahydropyridin-4-ylidene ammonium salt) reacts with aldehydes to a 2,3-dihydropyridin-4-one substituted at position 5, i.e., a compound of formula (I). Due to the enolate reactivity of the related enaminones (Niphakis MJ et al. J. Org. Chem. 2010, 75 6793-6805), it was rather expected that the aldol condensation occurs at the position 3 of the 2,3-dihydropyridine. However, it was surprisingly found that different aldehydes substituted the ring system at position 5 and induced a rearrangement of the exocyclic amine function.

Another aspect of the present invention relates to a compound of formula (I) 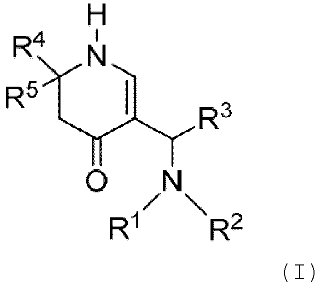 wherein R¹ and R² form together with the nitrogen to which they are bound a substituted or unsubstituted 5-, 6- or 7-membered heterocyclyl group, or
wherein R¹ and R² are independently from each other selected from the group consisting of hydrogen, unsubstituted or substituted C₁-C₁₀-alkyl, and unsubstituted or substituted C₆-C₁₄-aryl,
wherein R³ is selected from the group of unsubstituted or substituted C₁-C₁₀-alkyl, unsubstituted or substituted C₃-C₁₀-cycloalkyl, unsubstituted or substituted C₆-C₁₄-aryl, and unsubstituted or substituted 5-, 6- or 7-membered heteroaryl, and
wherein R⁴ and R⁵ are independently from each other a substituted or unsubstituted C₁-C₁₀-alkyl group,
wherein R⁴ is selected from the group consisting of substituted or unsubstituted C₁-C₁₀-alkyl, preferably a branched C₃-C₁₀-alkyl group, and unsubstituted or substituted C₃-C₁₀-cycloalkyl and R⁵ is hydrogen,
or wherein R⁴ and R⁵ form together with the carbon to which they are bound a substituted or unsubstituted C₃-C₁₀-cycloalkyl.

The 2,3-dihydropyridin-4-one compound of formula (I) of the present invention is substituted at position 5 with a disubstituted methyl group, wherein the first substituent of the methyl group is an amino-function and the second substituent is an alkyl, cycloalkyl, aryl or heteroaryl group. Furthermore, in the compound of formula (I) at least one of R⁴ or R⁵ is not hydrogen, preferably both of R⁴ and R⁵ are alkyl, such that the compound is a 2-substituted piperidin-4-one. Such a compound can be obtained with the process of the present invention.

Another aspect of the present invention relates to the compound of formula (I) for use as a medicament, in particular, for the use in the treatment or prevention of an infection, preferably a bacterial infection, or a cancer.

Disclosed is also a method of treating or preventing a disease, wherein the method comprises a step of administering the compound of formula (I) to a subject in need thereof. Particularly, the disease is an infection or a cancer.

### DESCRIPTION OF EMBODIMENTS

The compounds of formula (II) which are used to synthesize the compounds of formula (I) can be manufactured according to the state of the art as described for example in Seebacher W et al. (Monatshefte für Chemie 2015, 146, 1299-1308).

Preferably, the compound of formula (II) and the respective compound of formula (I) are N,N-dialkylated. Thus, preferably, R¹ and R² both are independently from each other substituted or unsubstituted C₁-C₁₀-alkyl group, such as methyl, and ethyl, preferably methyl, or R¹ and R² form together with the nitrogen to which they are bound a substituted or unsubstituted 5-, 6-, or 7-membered heterocyclyl group.

Preferably, both R¹ and R² are selected to result in a symmetric substitution of the nitrogen to which they are bound. Accordingly, in one embodiment of the present invention, R¹ and R² are identical to each other.

In an embodiment of the present invention, R¹ and R² form together with the nitrogen a non-aromatic heterocyclyl group. In particular, R¹ and R² form together with the nitrogen to which they a bound an aliphatic heterocyclyl group with 5 to 7 ring members, i.e., a substituted or unsubstituted 5-, 6-, or 7-membered heterocyclyl group.

For example, R¹ and R² form together with the nitrogen to which they are bound a heterocyclyl group selected from the group consisting of azepanyl, piperidinyl and pyrrolidinyl. Moreover, the heterocyclyl group can contain in addition to the nitrogen one or more heteroatoms selected from the group consisting of nitrogen, sulfur and oxygen, preferably sulphur and oxygen. The heterocyclyl group can be substituted by one or more substituent(s), preferably selected from the group consisting of amino, halide, cyano, hydroxyl, a C₁-C₄-alkoxy group, and a C₁-C₁₀-alkyl group, in particular amino and C₁-C₄-alkyl group.

In an embodiment of the present invention, R¹ and R² form together with the nitrogen to which they are bound a heterocyclyl group selected from the group consisting of azepanyl, piperidinyl, pyrrolidinyl and morpholinyl, preferably piperidinyl, pyrrolidinyl and morpholinyl.

The compound of formula (II) is preferably alkylated or dialkylated at position 2. The presence of alkyl groups at position 2 may have a beneficial influence on the stereoselectivity with the aldehyde of formula (III) due to steric hindrance of position 3.

In a preferred embodiment of the present invention, both of R⁴ and R⁵ are a substituted or unsubstituted C₁-C₁₀-alkyl group, more preferably an unsubstituted C₁-C₆-alkyl group. In a preferred embodiment, R⁴ and R⁵ are identical to each other.

In a preferred embodiment of the present invention, R⁴ and R⁵ both are methyl or ethyl, preferably both are methyl.

In another preferred embodiment of the present invention, R⁴ is a substituted or unsubstituted C₁-C₁₀-alkyl group and R⁵ is hydrogen. If R⁵ is hydrogen, preferably, R⁴ is a C₃-C₆-alkyl group, in particular, R⁴ is a branched C₃-C₆-alkyl group, such as for example isopropyl or cyclohexyl, preferably isopropyl.

In another embodiment of the present invention, R⁴ and R⁵ form together with the carbon to which they are bound a substituted or unsubstituted C₃-C₁₀-cycloalkyl. The corresponding spiro compound of formula (II) can be obtained analogously to the approach disclosed in Seebacher W et al. (Monatshefte für Chemie 2015, 146, 1299-1308).

The aldehyde of formula (III) as used in the process of the invention can be synthesized according to the state of the art. R³ is selected from the group of unsubstituted or substituted C₁-C₁₀-alkyl, unsubstituted or substituted C₃-C₁₀-cycloalkyl, unsubstituted or substituted C₆-C₁₄-aryl, and unsubstituted or substituted 5-, 6- or 7-membered heteroaryl.

An aldehyde of formula (III) with a cyclic or branched substituent as R³ is particularly preferred as steric influences could direct towards the reaction at position 5 of the compound of formula (II).

In a preferred embodiment of the invention, R³ is selected from the group of unsubstituted or substituted branched C₃-C₁₀-alkyl.

In another preferred embodiment of the invention, R³ is an unsubstituted or substituted 6-membered cycloalkyl, aryl or heteroaryl. In particular, R³ is selected from the group consisting of phenyl, pyridyl and cyclohexyl.

In further preferred embodiment of the invention, R³ is a C₆-aryl, in particular a C₆-aryl substituted in ring position 4, preferably selected from the group consisting of phenyl, 4-fluorphenyl, 4-isopropylphenyl, 4-methylphenyl, 2-trifluormethylphenyl, 4-pyridyl, cyclohexyl.

The term "substituted" refers to a chemical entity being substituted with one or more substituents. The term "one or more substituents" refers to from one to the maximum number of substituents possible, depending on the number of available substitution sites.

The term "aryl" as used herein in particular refers to a "C₅-C₁₄-aryl", i.e., an aromatic carbocyclic ring group having a specified number of carbon atoms. For example, C₅-C₆-aryl group refers to an aromatic ring having at least 5 and at most 6 carbon atoms. The term "aryl group" also refers to a multicyclic group having more than one aromatic ring, such as C₁₀-C₁₄-aryl group. Useful examples are cyclopentadienyl, phenyl, naphthyl, anthracyl and phenantryl. Typical substituted aryl groups are 4-chlorophenyl, 4-bromophenyl, 4-fluorophenyl, 4-trifluoromethylphenyl, 4-methylphenyl, 4-methoxyphenyl, 4-isopropylphenyl, 6-fluoro-naphthalen-1-yl, 6-chloro-naphtalen-1-yl, 6-methoxy-naphthalen-1-yl, 6-trifluoromethyl-naphthalen-1-yl, 3-methoxy-1-naphthalen-1-yl, 3-chloro-naphthalen-1-yl, 3-trifluoromethyl-naphthalen-1-yl, 3-fluoro-naphthalen-1-yl, 3,5-dichlorophenyl, 3,4-dichlorophenyl, 2-chlorophenyl, 2,6-dichlorophenyl, 3,5-difluoro-naphthalen-1-yl, 3,5-dichloronaphthalen-1-yl, 3,5-dimethoxy-naphthalen-1-yl, 3,5-bis(trifluoromethyl)naphthalen-1-yl, 4-nitrophenyl, and 4-aminophenyl.

The term "C₁-C₁₀-alkyl" as used herein refers to straight or branched hydrocarbon chain groups having a specified number of carbon atoms. For example, a C₁-C₁₀-alkyl group refers to a straight or branched alkyl group having at least 1 and at most 10 carbon atoms. Alkyl groups having a straight chain preferably represent C₁-C₇-alkyl. Useful branched alkyl groups, which preferably represent C₃-C₁₀-alkyl groups, are for example isopropyl, isobutyl, sec.-butyl, tert.-butyl, pentan-1-yl, pentan-2-yl, pentan-3-yl and 6-ethyloctan-2-yl.

The term "C₃-C₁₀-cycloalkyl" as used herein refers to cyclic hydrocarbon group having a specified number of carbon atoms. For example, a C₆-alkyl group refers to a cyclohexyl. Useful C₃-C₁₀-cycloalkyl groups include for example, cyclopentyl, cyclohexyl, cycloheptanyl, and cyclooctanyl.

The term "heteroaryl" as used herein refers to an aromatic group having one or more heteroatoms (O, S or N). A multicyclic group having one or more heteroatoms, wherein at least one ring of the group is heteroaromatic, is referred to as a "heteroaryl" as well. Typical substituted heteroaryl groups are 2-methylpyridin-4-yl, 3-methylpyridin-4-yl, 4-methylpyridin-3-yl, 5-methylpyridin-3-yl, 6-methylpyridin-3-yl, 2-methylpyridin-3-yl, 6-chloro-quinolin-1-yl, 6-fluoro-quinolin-1-yl, 6-methoxy-quinolin-1-yl, 6-trifluoromethyl-quinolin-1-yl, 7-chloro-quinolin-1-yl, 7-fluoro-quinolin-1-yl, 7-methoxy-quinolin-1-yl, 7-trifluoromethyl-quinolin-1-yl, 8-chloro-quinolin-1-yl, 8-fluoro-quinolin-1-yl, 8-methoxy-quinolin-1-yl, and 8-trifluoromethyl-quinolin-1-yl.

The term "heterocyclyl" as used herein refers to a non-aromatic group heterocyclyl groups having one or more heteroatoms (N, O or S) and a total number of ring members including carbon atoms and heteroatom(s). A multicyclic group having one or more heteroatoms, wherein at least one ring of the group is heterocyclic, is referred to as a "heterocyclyl" as well. Typical heterocyclyl groups include for example the nitrogen containing heterocyclyl groups azepanyl, piperidinyl, pyrrolidinyl, and morpholinyl.

The compound of formula (II) may be provided in the form of the free base or as a salt. A salt of a compound of formula (II) may be defined by formula (IV) 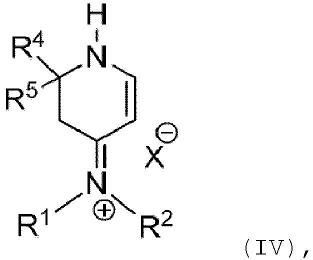 wherein X⁻ is an anion. A salt of formula (IV) is also referred to as tetrahydropyridin-4-ylidene ammonium salt. The anion may be conventionally selected as the reaction conditions typically induce the free base. In one embodiment of the present invention, X⁻ is a halide anion selected from the group consisting of F⁻, Cl⁻, Br⁻ and I⁻.

The compound of formula (I) has at least one stereocenter due to the chiral carbon atom representing the methyl substitution at position 5 of the compound of formula (I). In a compound of formula (I) both enantiomers, designated as R- and S-enantiomer, should be understood to be covered if the stereochemistry is not explicitly indicated.

In one embodiment of the present invention, the compound of formula (I) is selected from the group consisting of:
(RS)-2,3-Dihydro-2,2-dimethyl-5-(phenyl(pyrrolidin-1-yl) methyl)pyridin-4(1H)-one,
(RS)-2,3-Dihydro-2,2-dimethyl-5-(phenyl(piperidin-1-yl)methyl)pyridin-4(1H)-one,
(RS)-2,3-Dihydro-2,2-dimethyl-5-(phenyl(morpholin-1-yl)methyl)pyridin-4(1H)-one,
(RS)-2,3-Dihydro-2,2-dimethyl-5-((4-fluorphenyl)-(pyrrolidin-1-yl)methyl)pyridin-4(1H)-one,
(RS)-2,3-Dihydro-2,2-dimethyl-5-((4-isopropylphenyl)-(pyrrolidin-1-yl)methyl)pyridin-4(1H)-one,
(RS)-2,3-Dihydro-2,2-dimethyl-5-((4-pyridyl)-(pyrrolidin-1-yl)methyl)pyridin-4(1H)-one,
(RS)-2,3-Dihydro-2,2-dimethyl-5-((4-fluorphenyl) (morpholin-1-yl)methyl)pyridin-4(1H)-one,
(RS)-2,3-Dihydro-2,2-dimethyl-5-((4-methylphenyl)-(pyrrolidin-1-yl)methyl)pyridin-4(1H)-one,
(RS)-2,3-Dihydro-2,2-dimethyl-5-((pyrrolidin-1-yl)-(2-trifluormethylphenyl)methyl)pyridin-4(1H)-one,
(RS)-2,3-Dihydro-2,2-dimethyl-5-((4-fluorphenyl)-(piperidin-1-yl)methyl)pyridin-4(1H)-one,
(RS)-5-(Cyclohexyl(pyrrolidin-1-yl)methyl)-2,2-dimethyl-2,3-dihydropyridin-4(1H)-one, and
(RS)-5-(Cyclohexyl(piperidin-1-yl)methyl)-2,2-dimethyl-2,3-dihydropyridin-4(1H)-one.

In the process of the invention, the step of reacting the compound of formula (II) with the aldehyde of formula (III) is preferably performed in alkaline solution, more preferably in aqueous alkaline solution.

In a preferred embodiment of the present invention, the solvent for the reaction step (i.e., the step of reacting the compound of formula (II) with the aldehyde of formula (III)) is selected from water, low alcohols, such as ethanol, and mixtures thereof, preferably water. In one embodiment of the present invention, the pH of the solution is above 10, preferably above 12, more preferably above 13, in particular 14. Preferably, the solution contains a base. The base ensures a high pH value as specified above. In a preferred embodiment, the base is selected from the group consisting of tertiary amines, hydrocarbonate salts, carbonate salts, and hydroxide salts, preferably hydroxide salts, such as NaOH and KOH, more preferably KOH.

Preferably, for the reaction step, the compound of formula (II) and the aldehyde of formula (III) are provided in a molar ratio of about 1:1, e.g., 1:1.05 to 1:1.1

In one embodiment of the present invention, the reaction step is performed at room temperature for a reaction time of from 3 to 10 days, preferably 4, 5 or 6 days.

In one embodiment of the present invention, the process of the invention further comprises one or more steps of (re)crystallizing the compound of formula (I) to obtain it or a salt thereof in a crystalline form. Suitable solvents for (re)crystallization are for example ethyl acetate, diethyl ether, cyclohexane, ethanol, isopropanol, acetone, or mixtures thereof.

Accordingly, in one embodiment of the present invention, the present invention also relates to the compound of formula (I) in crystalline form.

In another embodiment of the present invention, the present invention also relates to the compound of formula (I) in the form of a salt thereof, in particular an acid addition salt of the compound of formula (I).

It was further found that compounds of formula (I) show antimicrobial activity, in particular antibacterial activity. Furthermore, the compounds showed a dose dependent cytotoxic effect on a human cancer cell line, in particular a leukaemia cell line. On the other hand, the cytotoxicity of the compounds towards mammalian cells, preferably human cells, was shown to be low. Hence, the compounds of the present invention can be administered safely to mammalians and humans and based on the findings should be useful in treatment and prevention of specific diseases, in particular (bacterial) infections or cancer.

Accordingly, in one embodiment of the present invention, the invention relates to the use of a compound of formula (I) in the treatment or prevention of an infection (i.e., a disease mediated by an infectious pathogen), preferably for use in treatment and prevention of a bacterial infection.

In another embodiment, the invention relates to the use of a compound of formula (I) in the treatment or prevention of a cancer, in particular a blood cancer, more particular leukaemia.

In a preferred embodiment of the present invention, the compound of formula (I) for use as a medicament is administered to a subject in need thereof. Preferably, the subject is a human or an animal.

The compound for use of the present invention or the compound as used in a method of treating or preventing a disease can be readily formulated to a pharmaceutical composition. A pharmaceutical composition preferably includes the compound of formula (I), preferably in a pharmaceutically effective amount, and at least one pharmaceutically acceptable excipient. The person of ordinary skill in the art may select a pharmaceutically acceptable excipient comprised in a composition out of the group consisting of appropriate carriers, diluents, e.g., including fillers, binders, disintegrants, flow conditioners, lubricants, fragrances, preservatives, stabilizers, wetting agents and/or emulsifiers, solubilizers, salts for regulating osmotic pressure and/or buffers based on the common knowledge in the pharmaceutical art. The person of ordinary skill in the art is well aware of factors influencing the appropriate choice of the pharmaceutically effective amount and suitable excipients including factors such as the subject to be treated, the disease, the desired effect (treatment or prevention), and the route of administration. For example, for treatment and prevention of local (bacterial) infections, topical application forms of the compound of formula (I) may be preferred including for example creams, lotions, transdermal patches, topical solutions, to be installed at the site of infection such as for example the eye, ear, nose, or a specific region of the skin including the mucous membranes. On the other hand, systemic application forms including enteral (e.g., oral) or parenteral application (e.g., intravenous) may be preferred for treatment or prevention of systemic (bacterial) infections or cancer, in particular non-solid tumors, such as blood cancer.

The present invention is further illustrated by the following examples, yet without being restricted thereto.

### EXAMPLES

The following abbreviations are used:
- CH: cyclohexane
- DMSO: dimethyl sulfoxide
- EtOAc: ethyl acetate
- IC₅₀: half maximal inhibitory concentration
- NMR: nuclear magnetic resonance spectroscopy
- m.p.: melting point
- r.t.: room temperature
- XTT: 2,3-Bis-(2-methoxy-4-nitro-5-sulfophenyl)-2H-tetrazolium-5-carboxanilide

### 2-Substituted 1,2,3,4-tetrahydropyridin-4-ylidene salts

The 2,2-dimethyl-2,3-dihydropyridin-4-amines are educts of the reactions in Examples 1-12. They were prepared via a selective hydrogenation of their methylthio compounds as reported (Seebacher W et al. Monatshefte für Chemie, 2015, 146, 1299-1308; DOI:10.1007/s00706-015-1503-y).

The compounds were yielded as salts and processed as such.

### Example 1: (RS)-2,3-Dihydro-2,2-dimethyl-5-(phenyl(pyrrolidin-1-yl)methyl)pyridin-4(1H)-one

2000 mg (6.53 mmol) of 1-(2,2-Dimethyl-1,2,3,4-tetrahydropyridin-4-ylidene)pyrrolidinium iodide were suspended in 16 ml of aqua dest. and 1466 mg (26.13 mmol) of KOH dissolved in 16 ml of aqua dest. were added. The mixture was stirred at r.t. until all of the salt was dissolved (ca. 5-10 minutes). To the resulting yellow solution 693 mg (6.53 mmol; 666 µl) of benzaldehyde (purified via a short Alox b column) were added. The reaction mixture was stirred for 4 days at r.t.. The separated crystalline solid was filtered with suction, washed clean with water and a few drops of acetone and dried *in vacuo* giving 1261 mg (68 %) of 2,3-dihydro-2,2-dimethyl-5-(phenyl(pyrrolidin-1-yl)methyl)pyridin-4(1H)-one as orange-yellow solid. For analytical purposes it was recrystallized from ethyl acetate/cyclohexane and washed clean with cyclohexane followed by a small amount of ethyl acetate giving a white solid.

m.p.(EtOAc/CH): 147-148°C

¹H NMR (CDCl₃, δ, 400 MHz) : 1.19 (s, 3H, CH₃), 1.29 (s, 3H, CH₃), 1.75 (br, s, 4H, 2CH₂), 2.37 (s, 2H, 3-H), 2.50 (br, s, 4H, 2NCH₂), 4.52 (s, 1H, CH), 4.60 (d, *J* = 5.5 Hz, 1H, 1-H), 7.14 - 7.38 (m, 6H, 6-H, ArH) ppm; ¹³C NMR (CDCl₃, δ, 100 MHz) : 23.49 (2CH₂), 26.57, 27.57 (2CH₃), 49.18 (C-3), 53.17 (2NCH₂), 53.80 (C-2), 63.98 (CHAr), 111.43 (C-5), 126.09, 127.14, 128.03 (5ArC) , 144.87 (ArC_{q}), 149.23 (C-6), 189.50 (C-4) ppm.

### Example 2: (RS)-2,3-Dihydro-2,2-dimethyl-5-(phenyl(piperidin-1-yl)methyl)pyridin-4(1H)-one

2091 mg (6.53 mmol) of 1-(2,2-Dimethyl-1,2,3,4-tetrahydropyridin-4-ylidene)piperidinium iodide were suspended in 16 ml of aqua dest. and 1470 mg (26.20 mmol) of KOH dissolved in 16 ml of aqua dest. were added. The mixture was stirred at r.t. until all of the salt was dissolved (ca. 10-20 minutes). To the resulting yellow solution 695 mg (7.71 mmol; 668 µl) of benzaldehyde (purified via a short Alox b column) were added. The reaction mixture was stirred for 5 days at r.t.. The separated crystalline solid was filtered with suction, washed clean with water and a few drops of acetone and dried in vacuo giving 972 mg (50%) of 2,3-dihydro-2,2-dimethyl-5-(phenyl(piperidin-1-yl)methyl)pyridin-4(1H)-one as an orange-yellow solid. For analytical purposes it was recrystallized from ethyl acetate giving a white solid.

m.p.(EtOAc): 158°C

¹H NMR (DMSO-d₆, δ, 400 MHz) : 1.09 (s, 3H, CH₃), 1.18 (s, 3H, CH₃), 1.31 - 1.51 (m, 6H, 3CH₂), 2.13 - 2.22. (m, 4H, 3-H, NCH₂), 2.28 - 2.38 (m, 2H, NCH₂), 4.37 (s, 1H, CHAr), 6.95 (d, *J* = 6.8 Hz, 1H, 6-H), 7.09 - 7.14 (m, 1H, ArH), 7.20 - 7.26 (m, 4H, ArH), 7.35 (d, *J* = 6.8 Hz, 1H, 1-H) ppm; ¹³C NMR (DMSO-d₆, δ, 100 MHz) : 24.62 (CH₂), 26.11 (2CH₂), 26.19, 26.78 (2CH₃), 48.90 (C-3), 52.34 (2NCH₂), 53.03 (C-2), 64.17 (CHAr), 106.75 (C-5), 125.98, 127.30, 128.17 (5ArC), 144.62 (ArC_{q}), 149.38 (C-6), 188.49 (C-4) ppm.

### Example 3: (RS)-2,3-Dihydro-2,2-dimethyl-5-(phenyl(morpholin-1-yl)methyl)pyridin-4(1H)-one

2000 mg (6.22 mmol) of 1-(2,2-Dimethyl-1,2,3,4-tetrahydropyridin-4-ylidene)morpholinium iodide were suspended in 30 ml of aqua dest. and 1.397 g (24.89 mmol) of solid KOH were added. To the resulting yellow solution 660 mg (6.22 mmol) of benzaldehyde (purified via a short Alox b column) were added. The reaction mixture was stirred for 4 days at r.t.. The separated crystalline solid was filtered with suction, washed clean with water and a few drops of acetone and dried over phosphorous pentaoxide giving 1.436 g (77%) of 2,3-dihydro-2,2-dimethyl-5-(phenyl(morpholin-1-yl)methyl)pyridin-4(1H)-one as an off-white solid. For analytical purposes it was recrystallized from ethyl acetate giving white needles. (0.595g, 32%) .

m.p. (EtOAc): 165°

¹H NMR (DMSO-d₆, δ, 400 MHz) : 1.09 (s, 3H, CH₃), 1.17 (s, 3H, CH₃), 2.15 - 2.25 (m, 4H, 3-H, NCH₂), 2.29 - 2.39 (s, 2H, NCH₂), 3.49 - 3.60 (br, s, 4H, 2OCH₂), 4.36 (s, 1H, CHAr), 7.00 (d, *J* = 6.8 Hz, 1H, 6-H), 7.13 - 7.16 (m, 1H, ArH), 7.25 - 2.26 (m, 4H, ArH), 7.42 (d, *J* = 6.9 Hz, 1H, 1-H) ppm; ¹³C NMR (DMSO-d₆, δ, 100 MHz): 26.20, 26.77 (2CH₃), 48.82 (C-3), 52.11 (2NCH₂), 53.04 (C-2), 64.25 (CHAr), 66.61 (2OCH₂), 106.04 (C-5), 126.26, 127.42, 128.33 (5ArC), 143.71 (ArC_{q}), 149.51 (C-6), 188.47 (C-4) ppm.

### Example 4: (RS)-2,3-Dihydro-2,2-dimethyl-5-((4-fluorphenyl)-(pyrrolidin-1-yl)methyl)pyridin-4(1H)-one

2000 mg (6.53 mmol) of 1-(2,2-Dimethyl-1,2,3,4-tetrahydropyridin-4-ylidene)pyrrolidinium iodide were suspended in 16 ml of aqua dest. and 1466 mg (26.13 mmol) of KOH dissolved in 16 ml of water were added. The mixture was stirred at r.t. until all of the salt was dissolved (ca. 5-10 minutes). To the resulting yellow solution 810 mg (6.53 mmol) of 4-fluorobenzaldehyde (purified via a short Alox b column) were added. The reaction mixture was stirred for 6 days at r.t.. The separated crystalline solid was filtered with suction, washed clean with water and dried *in vacuo* over phosphorous pentoxide giving 1.658 mg (84 %) of the product as beige solid. It was recrystallized from ethyl acetate and washed clean with an ice-cold mixture of ethyl acetate/cyclohexane giving white needles. Yield: 1.072 g, 54%

m.p. (EtOAc): 169°C

¹H NMR (DMSO-d₆, δ, 400 MHz) : 1.07 (s, 3H, CH₃), 1.16 (s, 3H, CH₃), 1.62 - 1.65 (m, 4H, 2CH₂), 2.14 (s, 2H, 3-H), 2.25 - 2.39 (m, 2H, NCH₂), 2.33 - 2.39 (m, 2H, NCH₂), 4.29 (s, 1H, CHAr), 7.00 - 7.05 (m, 2H, ArH), 7.10 (d, *J* = 6.8 Hz, 1H, 6-H), 7.25 - 7.29 (m, 2H, ArH), 7.36 (d, *J* = 6.9 Hz, 1H, 1-H) ppm; ¹³C NMR (DMSO-d₆, δ, 100 MHz) : 23.34 (2CH₂), 26.02, 26.85 (2CH₃), 48.92 (C-3), 52.79 (2NCH₂), 53.03 (C-2), 63.35 (CHAr), 108.89 (C-5), 114.74 (d, ²J(C,F) = 21.0 Hz, ArC), 128.66 (d, ³J(C,F) = 7.9 Hz, ArC), 141.72 (d, ⁴J(C,F) = 3.1 Hz, ArC_{q}), 149.08 (C-6), 160.69 (d, ¹J(C,F) = 241.3 Hz, ArC_{q}), 187.86 (C-4) ppm.

### Example 5: (RS)-2,3-Dihydro-2,2-dimethyl-5-((4-isopropylphenyl)-(pyrrolidin-1-yl)methyl)pyridin-4(1H)-one

2000 mg (6.53 mmol) of 1-(2,2-Dimethyl-1,2,3,4-tetrahydropyridin-4-ylidene)pyrrolidinium iodide were suspended in 32 ml of aqua dest. and 1466 mg (26.13 mmol) of KOH were added. The mixture was stirred at r.t. until all of the salt was dissolved (ca. 5-10 minutes) . To the resulting yellow solution 968 mg (6.53 mmol) of 4-isopropylbenzaldehyde (purified via a short Alox b column) were added. The reaction mixture was stirred for 5 days at r.t.. The separated resinous beige product was dried *in vacuo* over phosphorous pentoxide and then treated with hot ethyl acetate. The insoluble part was filtered off. Cyclohexane was added to the solution and it was allowed to stand overnight. The precipitate was filtered off and discarded and the filtrate evaporated to dryness and the residue was recrystallized from ethyl acetate giving a nearly pure product, a further crystallization from ethyl acetate afforded the pure product 118 mg (6%) as white needles.

m.p. (EtOAc) : 152°C

¹H NMR (DMSO-d₆, δ, 400 MHz): 1.09 (s, 3H, CH₃), 1.15 - 1.16 (m, 9H, CH (C*H*₃)₂, CH₃), 1.61 - 1.65 (m, 4H, 2CH₂), 2.14 (s, 2H, 3-H), 2.25 - 2.37 (m, 4H, 2NCH₂), 2.80 (quin, *J* = 6.9 Hz, 1H, C*H*(CH₃)₂), 4.28 (s, 1H, CHAr), 7.07 - 7.09 (m, 3H, 6-H, 2ArH), 7.16 (d, *J* = 8.0 Hz, 2H, 2ArH), 7.31 (d, *J* = 6.7 Hz, 1H, 1-H) ppm; ¹³C NMR (DMSO-d₆, δ, 100 MHz) : 23.33 (2CH₂), 24.09, 24.12 (CH(*C*H₃)₂), 25.94, 27.10 (2CH₃), 33.16 (CH(CH₃)₂), 48.95 (C-3), 52.86 (2NCH₂), 53.02 (C-2), 63.50 (CHAr), 109.23 (C-5), 125.99, 126.90 (4ArC), 142.98, 145.90 (ArC_{q}), 149.23 (C-6), 187.84 (C-4) ppm.

### Example 6: (RS)-2,3-Dihydro-2,2-dimethyl-5-((4-fluorphenyl)(morpholin-1-yl)methyl)pyridin-4 (1H) -one

700mg (2.18 mmol) of 1-(2,2-dimethyl-1,2,3,4-tetrahydropyridin-4-ylidene)morpholinium iodide were suspended in 11 ml of aqua dest. and 0.489 mg (8.71 mmol) of solid KOH were added. To the resulting yellow solution 271 mg (2.18 mmol) of 4-fluorbenzaldehyde (purified via a short Alox b column) were added. The reaction mixture was stirred for 6 days at r.t.. The separated resin was washed with water and dried in vacuo. It was triturated with hot ethyl acetate and the insoluble parts were filtered off and a part of the solvent removed by evaporation. The formed amorphous precipitate was filtered off and the filtrate concentrated in vacuo. The product crystallized in form of white needles, was sucked off and washed with cold ethyl acetate and dried. Yield.: 225 mg (32%).

m.p. (EtOAc): 162°C

¹H NMR (DMSO-d₆, δ, 400 MHz) : 1.09 (s, 3H, CH₃), 1.17 (s, 3H, CH₃), 2.14 - 2.23 (m, 4H, 3-H, NCH₂), 2.30 - 2.39 (m, 2H, NCH₂), 3.51 - 3.56 (m, 4H, 2OCH₂), 4.35 (s, 1H, CHAr), 7.00 (d, *J* = 6.8 Hz, 1H, 6-H), 7.07 (t, *J* = 8.8 Hz, 2H, ArH), 7.27 (dd, *J* = 8.5, 5.8 Hz, 2H, ArH), 7.45 (d, *J* = 6.9 Hz, 1H, 1-H) ppm; ¹³C NMR (DMSO-d₆, δ, 100 MHz): 26.18, 26.73 (2CH₃), 48.82 (C-3), 52.02 (2NCH₂), 53.05 (C-2), 63.72 (CHAr), 66.60 (2OCH₂), 105.78 (C-5), 114.99 (d, ²J(C,F) = 21.0 Hz, ArC), 129.15 (d, ³J(C,F) = 7.7 Hz, ArC), 139.72 (d, ⁴J(C,F) = 3.0 Hz, ArC_{q}), 149.46 (C-6), 160.81 (d, ¹J(C,F) = 241.8 Hz, ArC_{q}), 188.51 (C-4) ppm.

### Example 7: (RS)-2,3-Dihydro-2,2-dimethyl-5-((4-methylphenyl)-(pyrrolidin-1-yl)methyl)pyridin-4(1H)-one

1000 mg (3.27 mmol) of 1-(2,2-dimethyl-1,2,3,4-tetrahydropyridin-4-ylidene)pyrrolidinium iodide were suspended in 16 ml of aqua dest. and 733 mg (13.1 mmol) of KOH was added. The mixture was stirred at r.t. until all of the salt was dissolved (ca. 5-10 minutes) . To the resulting yellow solution 393 mg (3.27 mmol) of p-tolylaldehyde (purified via a short Alox b column) were added. The reaction mixture was stirred for 6 days at r.t.. The separated beige crystalline solid was filtered with suction, washed clean with water and dried *in vacuo* over phosphorous pentaoxide. It was treated with hot ethyl acetate, the insoluble parts removed by filtration and the filtrate concentrated *in vacuo.* Crystallization took place overnight giving the product as colorless plates. Yield: 440 mg (45%).

m.p. (EtOAc): 167°C

¹H NMR (DMSO-d₆, δ, 400 MHz) : 1.07 (s, 3H, CH₃), 1.16 (s, 3H, CH₃), 1.57 - 1.67 (m, 4H, 2CH₂), 2.09 - 2.17 (m, 2H, 3-H), 2.22 (s, 3H, ArCH₃), 2.24 - 2.40 (m, 4H, 2NCH₂), 4.27 (s, 1H, CHAr), 7.02 (d, *J* = 7.8 Hz, 2H, ArH), 7.08 (d, *J* = 6.8 Hz, 1H, 6-H), 7.13 (d, *J* = 7.8 Hz, 2H, ArH), 7.30 (d, *J* = 6.8 Hz, 1H, 1-H) ppm; ¹³C NMR (DMSO-d₆, δ, 100 MHz) : 20.79 (ArCH₃), 23.35 (2CH₂), 26.04, 26.92 (2CH₃), 48.98 (C-3), 52.81 (2NCH₂), 53.01 (C-2), 63.58 (CHAr), 109.27 (C-5), 126.91, 128.69 (ArC), 134.90, 142.63 (ArC_{q}), 149.13 (C-6), 187.83 (C-4) ppm.

### Example 8: (RS)-2,3-Dihydro-2,2-dimethyl-5-((pyrrolidin-1-yl)-(2-trifluormethylphenyl)methyl)pyridin-4(1H)-one

1000 mg (3.27 mmol) of 1-(2,2-dimethyl-1,2,3,4-tetrahydropyridin-4-ylidene)pyrrolidinium iodide were suspended in 16 ml of aqua dest. and 733 mg (13.1 mmol) of KOH was added. The mixture was stirred at r.t. until all of the salt was dissolved (ca. 5-10 minutes) . To the resulting yellow solution 569 mg (3.27 mmol) of 2-(trifluoromethyl)benzaldehyde (purified via a short Alox b column) were added. The reaction mixture was stirred for 4 days at r.t.. The separated resinous solid was washed with water and dried *in vacuo* over phosphorous pentaoxide. It was dissolved in hot ethylacetate and cyclohexane was added till first turbidity appeared. An oil separated which solidified. This amorphous precipitate was filtered off and discarded. The filtrate was evaporated and dissolved in hot ethyl acetate. It was allowed to stand for some days till crystallization took place giving the product as white needles. Yield: 282 mg (24%).

m.p. (EtOAc): 139°C

¹H NMR (DMSO-d₆, δ, 400 MHz) : 1.08 (s, 3H, CH₃), 1.14 (s, 3H, CH₃), 1.58 - 1.67 (m, 4H, 2CH₂), 2.12 - 2.21 (m, 2H, 3-H), 2.28 - 2.46 (m, 4H, 2NCH₂), 4.81 (s, 1H, CHAr), 6.70 (d, *J* = 6.8 Hz, 1H, 6-H), 7.25 (d, *J* = 6.9 Hz, 1H, 1-H), 7.38 (t, *J* = 7.6 Hz, 1H, ArH), 7.60 (d, *J* = 7.9 Hz, 1H, ArH), 7.64 (t, *J* = 7.6 Hz, 1H, ArH), 8.04 (d, *J* = 7.8 Hz, 1H, ArH) ppm; ¹³C NMR (DMSO-d₆, δ, 100 MHz) : 23.31 (2CH₂), 26.33, 26.74 (2CH₃), 48.89 (C-3), 52.05 (2NCH₂), 52.95 (C-2), 57.93 (CHAr), 107.40 (C-5), 124.46 (q, ¹J(C,F) = 274 Hz, CF₃), 125.93 (q, ³J(C,F) = 6.1 Hz, ArC), 125.99 (q, ²J(C,F) = 29.5 Hz, ArC_{q}), 126.68 (ArC), 129.67 (ArC), 132.26 (ArC), 143.16 (ArC_{q}), 149.61 (C-6), 187.94 (C-4) ppm.

### Example 9: (RS)-2,3-Dihydro-2,2-dimethyl-5-((4-pyridyl)-(pyrrolidin-1-yl)methyl)pyridin-4(1H)-one

2000 mg (6.53 mmol) of 1-(2,2-Dimethyl-1,2,3,4-tetrahydropyridin-4-ylidene)pyrrolidinium iodide were suspended in 32 ml of aqua dest. and 1466 mg (26.13 mmol) of KOH were added. The mixture was stirred at r.t. until all of the salt was dissolved (ca. 5-10 minutes). To the resulting yellow solution 700 mg (6.53 mmol) of 4-pridinecarboxaldehyde (purified via a short Alox b column) were added. The reaction mixture was stirred for 4 days at r.t.. The yellow solution was extracted 5 times with dichloromethane and the combined organic layers were dried over sodium sulfate, filtered and the solvents evaporated *in vacuo* giving a yellow oil. This was dissolved in hot ethyl acetate cooled to r.t. and cyclohexane was added till the turbidity remains. After stirring overnight, the precipitate was sucked off and dissolved in hot ethyl acetate, filtered from insoluble parts and the solution left for crystallization giving 170 mg (9%) of the product as slightly yellowish needles.

m.p. (EtOAc): 169°C

¹H NMR (DMSO-d₆, δ, 400 MHz) : 1.07 (s, 3H, CH₃), 1.16 (s, 3H, CH₃), 1.64 - 1.67 (m, 4H, 2CH₂), 2.16 (s, 2H, 3-H), 2.28 - 2.50 (m, 4H, 2NCH₂), 4.33 (s, 1H, CHAr), 7.10 (d, *J* = 6.8 Hz, 1H, 6-H), 7.24 (dd, *J* = 4.5, 1.6 Hz, 2H, ArH), 7.49 (d, *J* = 6.9 Hz, 1H, 1-H), 8.40 (dd, *J* = 4.4, 1.6 Hz, 2H, ArH) ppm; ¹³C NMR (DMSO-d₆, δ, 100 MHz) : 23.34 (2CH₂), 26.01, 26.76 (2CH₃), 48.76 (C-3), 52.56 (2NCH₂), 53.08 (C-2), 63.14 (CHAr), 107.57 (C-5), 122.24 (ArC), 149.49, 149.57 (C-6, ArC), 154.17 (ArC_{q}), 187.77 (C-4) ppm.

### Example 10: (RS)-2,3-Dihydro-2,2-dimethyl-5-((4-fluorphenyl)-(piperidin-1-yl)methyl)pyridin-4(1H)-one

2091 mg (6.53 mmol) of 1-(2,2-dimethyl-1,2,3,4-tetrahydropyridin-4-ylidene)piperidinium iodide were suspended in 30 ml of aqua dest. and 1470 mg (26.20 mmol) of KOH were added. To the emulsion 810 mg (6.53 mmol) of 4-fluorobenzaldehyde (purified via a short Alox b column) were added. The reaction mixture was stirred for 4 days at r.t.. The separated crystalline solid was filtered with suction, washed clean with water and dried in vacuo over phosphorous pentoxide. It was dissolved in hot ethyl acetate and the insoluble part filtered off. The product crystallized overnight as needles, was sucked off and washed with ice cold ethyl acetate giving yellowish needles. Yield: 1.137 g, 55%.

m.p.(EtOAc): 183°C

¹H NMR (DMSO-d₆, δ, 400 MHz) : 1.10 (s, 3H, CH₃), 1.18 (s, 3H, CH₃), 1.34 - 1.48 (m, 6H, 3CH₂), 2.14 - 2.23 (m, 4H, 3-H, NCH₂), 2.26 - 2.36 (m, 2H, NCH₂), 4.37 (s, 1H, CHAr), 6.95 (d, *J* = 6.8 Hz, 1H, 6-H), 7.05 (t, *J* = 8.9 Hz, 2H, ArH), 7.25 (dd, *J* = 8.5, 5.8 Hz, 2H, ArH), 7.38 (d, *J* = 6.8 Hz, 1H, 1-H) ppm; ¹³C NMR (DMSO-d₆, δ, 100 MHz) : 24.56 (CH₂), 26.09 (2CH₂), 26.15, 26.72 (2CH₃), 48.88 (C-3), 52.22 (2NCH₂), 53.02 (C-2), 63.60 (CHAr), 106.41 (C-5), 114.78 (d, ²J(C,F) = 21.0 Hz, ArC), 128.96 (d, ³J(C,F) = 7.8 Hz, ArC), 140.55 (d, ⁴J(C,F) = 3.0 Hz, ArC_{q}), 149.31 (C-6), 160.66 (d, ¹J(C,F) = 241.6 Hz, ArC_{q}), 188.51 (C-4) ppm.

### Example 11: (RS)-5-(Cyclohexyl(pyrrolidin-1-yl)methyl)-2,2-dimethyl-2,3-dihydropyridin-4(1H)-one

2000 mg (6.53 mmol) of 1-(2,2-dimethyl-1,2,3,4-tetrahydropyridin-4-ylidene)pyrrolidinium iodide and 1501 mg (26.75 mmol) of KOH were suspended 30 ml of aqua dest. The mixture was stirred and sonicated at r.t. until nearly all was dissolved. To the resulting yellow solution 749 mg (6.68 mmol) of cyclohexyl carbaldehyde (purified via a short Alox b column) were added. The reaction mixture was stirred for 7 days at r.t.. The separated white precipitate was filtered with suction, washed clean with water and dried *in vacuo* giving a white solid. This was treated with dichloromethane and filtered. The filtrate was evaporated and the residue recrystallized repeatedly in from hot ethyl acetate giving 68 mg (4%) as colorless sparkling prisms.

m.p. (EtOAc) : 161°C

¹H NMR (DMSO-d₆, δ, 400 MHz) : 0.62 - 0.78 (m, 2H, CH₂), 0.95 - 1.02 (m, 1H, CH₂), 1.05 - 1.20 (m, 7H, CH₂, 2CH₃), 1.52 - 1.71 (m, 11H, CH, CH₂), 2.18 (br, s, 2H, 3-H), 2.22 - 2.26 (m, 2H, NCH₂), 2.28 - 2.33 (m, 2H, NCH₂), 3.23 (d, *J* = 6.5 Hz, 1H, CHN), 6.95 (d, *J* = 6.6 Hz, 1H, 6-H), 7.20 (d, *J* = 6.7 Hz, 1H, 1-H) ppm; ¹³C NMR (DMSO-d₆, δ, 100 MHz) : 22.99 (2CH₂), 26.03 (CH₂), 26.10 (CH₃), 26.26 (CH₂), 26.55 (CH₃), 26.83, 27.79, 31.26 (3CH₂), 40.45 (CH), 49.13 (C-3), 50.45 (2NCH₂), 52.78 (C-2), 61.81 (CHN), 103.50 (C-5), 149.44 (C-6), 189.39 (C-4) ppm.

### Example 12: (RS)-5-(Cyclohexyl(piperidin-1-yl)methyl)-2,2-dimethyl-2,3-dihydropyridin-4(1H)-one

2091 mg (6.53 mmol) of 1-(2,2-dimethyl-1,2,3,4-tetrahydropyridin-4-ylidene)piperidinium iodide and 1449 mg (25.82 mmol) of KOH were suspended 30 ml of aqua dest. The mixture was stirred and sonicated at r.t. until nearly all was dissolved. To the resulting yellow emulsion 749 mg (6.68 mmol) of cyclohexyl carbaldehyde (purified via a short Alox b column) were added. The reaction mixture was stirred for 7 d at r.t.. The separated off-white solid was filtered with suction, washed clean with water and dried. It was treated with dichloromethane and the suspension filtered. The filtrate was evaporated, and the residue dissolved in hot ethyl acetate. After a first crystallization, the solid was sucked off and from the filtrate crystallized the pure product in form of big yellowish prisms. Yield: 170 mg (9%) .

m.p. (EtOAc): 154°C

¹H NMR (DMSO-d₆, δ, 400 MHz): 0.57 - 0.66 (m, 1H, CH₂), 0.74 - 0.84 (m, 1H, CH₂), 1.00 - 1.16 (m, 3H, CH₂), 1.18, 1.19 (2s, 6H, 2CH₃), 1.22 - 1.75 (m, 11H, CH, CH₂), 1.92 - 2.00 (m, 1H, CH₂), 2.00 - 2.09 (m, 2H, NCH₂), 2.19 (s, 2H, 3-H), 2.22 - 2.29 (m, 2H, NCH₂), 3.14 (br, s, 1H, CHN), 6.90 (d, *J* = 6.6 Hz, 1H, 6-H), 7.18 (d, *J* = 6.6 Hz, 1H, 1-H) ppm; ¹³C NMR (DMSO-d₆, δ, 100 MHz) : 24.95 (CH₂), 25.84 (CH₃), 25.96, 26.03 (2CH₂), 26.35 (2CH₂), 26.65 (CH₃), 26.78, 30.50, 31.33 (3CH₂), 36.58 (CH), 49.14 (C-3), 50.24 (2NCH₂), 52.77 (C-2), 64.45 (CHN), 101.84 (C-5), 148.69 (C-6), 190.15 (C-4) ppm.

### Cytotoxicity assay using MRC-5 cells

Assays were performed using non-cancer human lung fibroblasts (MRC-5) and a colorimetric determination of cell viability using the tetrazolium salt XTT as reported ((Moshin N et al. Monatshefte für Chemie 2018, 149, 801-812). The anticancer drug vinblastine, the solvent DMSO and the untreated cells are used as control. The compounds of examples 1 to 8 were investigated in concentrations of 5 µM and 50 µM.

**Table 1: Results for cytotoxicity against MRC-5 cells**

| **Control** | | | | |
|---|---|---|---|---|
| | mean | S.E.M | | |
| DMSO 0.1 % | 100.00 | 5.36 | | |
| Vinblastine 100 nM | 51.88 | 5.01 | | |
| untreated | 105.47 | 3.62 | | |

| **Compounds** | **50 µM** | | **5 µM** | |
|---|---|---|---|---|
| | mean | S.E.M | mean | S.E.M |
| Example 1 | 87.86 | 9.76 | 107.18 | 3.97 |
| Example 2 | 82.27 | 2.56 | 108.05 | 3.80 |
| Example 3 | 84.05 | 1.45 | 110.88 | 4.96 |
| Example 4 | 90.99 | 1.26 | 105.92 | 2.42 |
| Example 5 | 42.19 | 4.85 | 104.11 | 3.56 |
| Example 6 | 89.35 | 2.74 | 105.36 | 1.95 |
| Example 7 | 88.41 | 1.39 | 100.07 | 4.67 |
| Example 8 | 67.08 | 3.08 | 103.65 | 0.68 |

The results are expressed as cell metabolic activity in % of the solvent control. The data show that the investigated compounds of the present invention have no effect on the cell viability at concentrations of 5 µM. At higher concentrations (50 µM) cell viability was better for most compounds when compared to the control substance vinblastine.

### Anticancer activity by cytotoxicity to human CCRF-CEM leukemia cells

Assays were performed using an established cancer cell line, human CCRF-CEM leukemia cells and cell viability determined as described above with a colorimetric assay using the tetrazolium salt XTT as reported (Moshin N et al. Monatshefte für Chemie 2018, 149, 801-812). The anticancer drug vinblastine, the solvent DMSO and the untreated cells are used as control. The compounds of examples 1 to 8 were investigated in concentrations of 5 µM and 50 µM.

**Table 2: Results for anticancer activity against CCRF-CEM cells expressed as cell metabolic activity in % of the solvent control**

| **Control** | | | | |
|---|---|---|---|---|
| | **mean** | **S.E.M** | | |
| DMSO 0.1 % | 100.00 | 1.95 | | |
| Vinblastine 100 nM | 9.06 | 0.16 | | |
| untreated | 104.95 | 1.66 | | |

| **Compounds** | **50 µM** | | **5 µM** | |
|---|---|---|---|---|
| | mean | S.E.M | mean | S.E.M |
| Example 1 | 9.02 | 0.24 | 88.69 | 1.51 |
| Example 2 | 8.75 | 0.07 | 88.18 | 0.82 |
| Example 3 | 10.98 | 0.26 | 93.13 | 1.93 |
| Example 4 | 8.82 | 0.18 | 88.03 | 1.63 |
| Example 5 | 8.72 | 0.14 | 75.36 | 1.58 |
| Example 6 | 8.78 | 0.11 | 87.71 | 2.93 |
| Example 7 | 8.52 | 0.12 | 87.47 | 2.35 |
| Example 8 | 8.46 | 0.11 | 52.06 | 2.02 |

The results are expressed as cell metabolic activity in % of the solvent control. The data show that the cytotoxic effect is in the same order of magnitude as for the anticancer drug vinblastine, if the compounds of the present invention are used at a concentration of 50 µM. Thus, the compounds of the present invention show a substantial anticancer activity.

### Antimicrobial activity

A drop plate method assay (Alajlani M et al. Chromatographia 2016, 79, 1527-1532.) with modification was performed to detect the antimicrobial activity against several bacteria strains. Investigated compounds (compounds of examples 1-7, 9) were dissolved in DMSO to a concentration of 1 mg/ml. Using sterile micropipette 10 µl of each compound was directly but gently dropped over seeded agar plate with test organism. The liquid was allowed to diffuse before the plate was inverted and incubated. The growth conditions for every strain were considered. The results were noted when a lawn of the indicator organism appeared on the plate (approximately 10-16 h).

The magnitude of activity was noted as high activity (++), medium activity (+), low activity (±) and no activity(-). Not determined data are indicated with n.d.

**Table 3: Antimicrobial activities of compounds_were detected over the test organisms**

| **Compound** | **Test Organism** | | | |
|---|---|---|---|---|
| | *Escherichia coli* | *Serratia sp* | *Micrococcus luteus* | *Pseudomonas fluorescens* |
| Example 1 | + | ± | + | + |
| Example 2 | + | ± | + | + |
| Example 3 | + | ± | ++ | + |
| Example 4 | ++ | + | + | + |
| Example 5 | + | ± | n.d. | ++ |
| Example 6 | + | ± | n.d. | ++ |
| Example 7 | + | ± | n.d. | ++ |
| Example 9 | + | ± | + | n.d. |

The investigated compounds inhibited the growth of both gram-negative *(E. coli)* and gram-positive (e.g., *Pseudomonas fluorescens*) bacteria at the investigated concentration.

## Claims

1. Process for preparing a compound of formula (I) 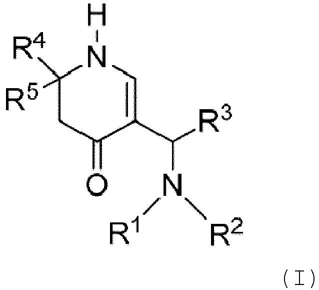 comprising a step of reacting a compound of formula (II) 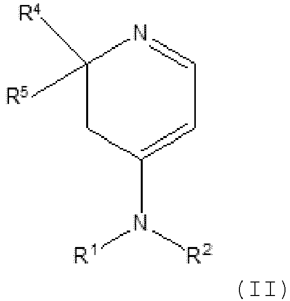 or a salt thereof
with an aldehyde compound of formula (III)
R³-CHO (III)
wherein R¹ and R² form together with the nitrogen to which they are bound a substituted or unsubstituted 5-, 6- or 7-membered heterocyclyl group, or
wherein R¹ and R² are independently from each other selected from the group consisting of hydrogen, unsubstituted or substituted C₁-C₁₀-alkyl, and unsubstituted or substituted C₆-C₁₄-aryl,
wherein R³ is selected from the group of unsubstituted or substituted C₁-C₁₀-alkyl, unsubstituted or substituted C₃-C₁₀-cycloalkyl, unsubstituted or substituted C₆-C₁₄-aryl, and unsubstituted or substituted 5-, 6- or 7-membered heteroaryl, and
wherein R⁴ and R⁵ are independently from each other a substituted or unsubstituted C₁-C₁₀-alkyl group,
wherein R⁴ is selected from the group consisting of substituted or unsubstituted C₁-C₁₀-alkyl, preferably a branched C₃-C₁₀-alkyl group, and unsubstituted or substituted C₃-C₁₀-cycloalkyl and R⁵ is hydrogen,
or wherein R⁴ and R⁵ form together with the carbon to which they are bound a substituted or unsubstituted C₃-C₁₀-cycloalkyl.

2. Process according to claim 1, wherein the reaction step is performed in alkaline solution, preferably in aqueous alkaline solution.

3. Process according to claim 1 or 2, further comprising a step of isolating and (re-)crystallizing the compound of formula (I).

4. A compound of formula (I) 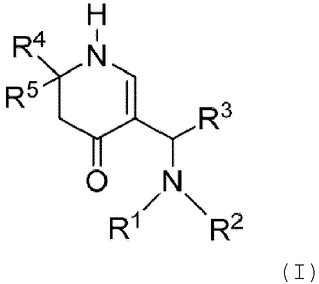
wherein R¹ and R² form together with the nitrogen to which they are bound a substituted or unsubstituted 5-, 6- or 7-membered heterocyclyl group, or
wherein R¹ and R² are independently from each other selected from the group consisting of hydrogen, unsubstituted or substituted C₁-C₁₀-alkyl, and unsubstituted or substituted C₆-C₁₄-aryl,
wherein R³ is selected from the group of unsubstituted or substituted C₁-C₁₀-alkyl, unsubstituted or substituted C₃-C₁₀-cycloalkyl, unsubstituted or substituted C₆-C₁₄-aryl, and unsubstituted or substituted 5-, 6- or 7-membered heteroaryl, and
wherein R⁴ and R⁵ are independently from each other a substituted or unsubstituted C₁-C₁₀-alkyl group,
wherein R⁴ is selected from the group consisting of substituted or unsubstituted C₁-C₁₀-alkyl, preferably a branched C₃-C₁₀-alkyl group, and unsubstituted or substituted C₃₋C₁₀-cydoalkyl and R⁵ is hydrogen,
or wherein R⁴ and R⁵ form together with the carbon to which they are bound a substituted or unsubstituted C₃-C₁₀-cydoalkyl.

5. Process according to any one of claims 1 to 3 or compound according to claim 4, wherein R¹ and R² form together with the nitrogen to which they are bound a heterocyclyl group selected from the group consisting of azepanyl, piperidinyl, pyrrolidinyl and morpholinyl, preferably piperidinyl, pyrrolidinyl and morpholinyl.

6. Process according to any one of claims 1 to 3 or 5, or compound according to claim 4 or 5, wherein R³ is selected from the group of unsubstituted or substituted branched C₃-C₁₀-alkyl.

7. Process according to any one of claims 1 to 3 or 5, or compound according to claim 4 or 5, wherein R³ is an unsubstituted or substituted 6-membered cycloalkyl, aryl or heteroaryl.

8. Process or compound according to claim 7, wherein R³ is selected from the group consisting of phenyl, pyridyl and cyclohexyl.

9. Process or compound according to claim 8, wherein R³ is selected from the group consisting of phenyl, 4-fluorphenyl, 4-isopropylphenyl, 4-methylphenyl, 2-trifluormethylphenyl, 4-pyridyl, cyclohexyl.

10. Process according to any one of claims 1 to 3 or 5 to 9, or compound according to claim 4 to 9, wherein R⁴ and R⁵ both are methyl.

11. Process according to claim 1 or compound according to claim 4, wherein the compound of formula (I) is selected from the group consisting of
(RS)-2,3-Dihydro-2,2-dimethyl-5-(phenyl(pyrrolidin-1-yl)methyl)pyridin-4(1H)-one,
(RS)-2,3-Dihydro-2,2-dimethyl-5-(phenyl(piperidin-1-yl)methyl)pyridin-4(1H)-one,
(RS)-2,3-Dihydro-2,2-dimethyl-5-(phenyl(morpholin-1-yl)methyl)pyridin-4(1H)-one,
(RS)-2,3-Dihydro-2,2-dimethyl-5-((4-fluorphenyl)-(pyrrolidin-1-yl)methyl)pyridin-4(1H)-one,
(RS)-2,3-Dihydro-2,2-dimethyl-5-((4-isopropylphenyl)-(pyrrolidin-1-yl)methyl)pyridin-4(1H)-one,
(RS)-2,3-Dihydro-2,2-dimethyl-5-((4-pyridyl)-(pyrrolidin-1-yl)methyl)pyridin-4(1H)-one,
(RS)-2,3-Dihydro-2,2-dimethyl-5-((4-fluorphenyl) (morpholin-1-yl)methyl)pyridin-4(1H)-one,
(RS)-2,3-Dihydro-2,2-dimethyl-5-((4-methylphenyl)-(pyrrolidin-1-yl)methyl)pyridin-4(1H)-one,
(RS)-2,3-Dihydro-2,2-dimethyl-5-((pyrrolidin-1-yl)-(2-trifluormethylphenyl)methyl)pyridin-4(1H)-one,
(RS)-2,3-Dihydro-2,2-dimethyl-5-((4-fluorphenyl)-(piperidin-1-yl)methyl)pyridin-4(1H)-one,
(RS)-5-(Cyclohexyl(pyrrolidin-1-yl)methyl)-2,2-dimethyl-2,3-dihydropyridin-4(1H)-one, and
(RS)-5-(Cyclohexyl(piperidin-1-yl)methyl)-2,2-dimethyl-2,3-dihydropyridin-4(1H)-one.

12. Compound according to any one of claims 4 to 11 for use as a medicament.

13. Compound according to any one of claims 4 to 11 for use in the treatment or prevention of an infection, preferably a bacterial infection, or a cancer.
